# EUROPEAN PATENT APPLICATION

(11) **EP 4 287 213 A1**
(43) Date of publication of application: **06.12.2023**
(21) Application number: 23708379.5
(22) Date of filing: 04.01.2023
(51) Int. Cl.: G16H 50/50, G16H 50/20, G16H 10/60, G16B 20/20, C12Q 1/6886

(54) **METHOD FOR PREDICTING CANCER RECURRENCE USING PATIENT-SPECIFIC PANEL**

(30) Priority: 29.03.2022 KR 20220038856
(71) Applicant: IMB DX, Inc., Geumcheon-gu Seoul 08506 (KR)
(72) Inventor: HEO, Sung Hoon, Ulsan 44644 (KR); LEE, Wook Jae, Seoul 06310 (KR); KIM, Su Yeon, Daejeon 34191 (KR); KIM, Hwang Phill, Gyeonggi-do 16990 (KR); MOON, Sung Tae, Seoul 05224 (KR); KIM, Tae You, Seoul 03009 (KR)
(74) Representative: Cabinet Nony
(86) International application number: PCT/KR2023/000146
(87) International publication number: WO 2023/191262

(57) **Abstract**

The present invention relates to a method of providing information for predicting cancer recurrence and prognosis using a patient-specific panel. According to the method for predicting cancer recurrence according to one embodiment of the present invention, it is possible to predict cancer recurrence and prognosis with increased sensitivity and accuracy.

## Description

### Technical Field

The present invention relates to a method of predicting cancer recurrence using a patient-specific panel.

### Background Art

As interest in cancer has increased, the survival rate of cancer patients has increased due to early diagnosis through active health examination, newly developed imaging technology, chemotherapy, and the like. However, more than 300 of patients who underwent surgery and received chemotherapy die due to cancer recurrence. Therefore, determining the optimal treatment by selectively identifying patients who are expected to have or suspected of having recurrence is very important to increase the survival rate of cancer patients.

In general, cancer recurrence is difficult to detect unless cancer grows to a size detectable by an imaging scan such as a CT scan. When cancer reaches a size detectable by imaging, cancer treatment is complicated and treatment options are limited. Therefore, there is a need for a highly sensitive and accurate method for early prediction of cancer recurrence, which is capable of predicting cancer recurrence.

Accordingly, the present inventors propose a method capable of predicting cancer recurrence by obtaining patient-specific genomic variant information from cancer tissue samples after surgery, generating a patient-specific panel using the information, and comparing genomic information for patient blood-derived samples.

### DISCLOSURE

### Technical Problem

An object of one aspect of the present invention is to provide a method for providing information for predicting cancer recurrence and prognosis, which is performed by a system for providing information for predicting cancer recurrence and prognosis, the method comprising steps of: a) obtaining patient-specific genomic variant information from a tissue sample from a patient of interest; b) generating a patient-specific panel by combining the obtained patient-specific genomic variant information and clinically actionable target information; and c) comparing information of the patient-specific panel with sequencing information of a blood-derived sample from the patient of interest, thereby determining whether the patient of interest has cancer recurrence.

An object of another object of the present invention is to provide a method for providing information for predicting cancer recurrence and prognosis, the method comprising steps of: a) obtaining patient-specific genomic variant information from a tissue sample from a patient of interest; b) generating a patient-specific panel by combining the obtained patient-specific genomic variant information and clinically actionable target information; and c) comparing information of the patient-specific panel with sequencing information of a blood-derived sample from the patient of interest, thereby determining whether the patient of interest has cancer recurrence.

### Technical Solution

One aspect of the present invention provides a method for providing information for predicting cancer recurrence and prognosis, which is performed by a system for providing information for predicting cancer recurrence and prognosis, the method comprising steps of: a) obtaining patient-specific genomic variant information from a tissue sample from a patient of interest; b) generating a patient-specific panel by combining the obtained patient-specific genomic variant information and clinically actionable target information; and c) comparing information of the patient-specific panel with sequencing information of a blood-derived sample from the patient of interest, thereby determining whether the patient of interest has cancer recurrence.

In one embodiment of the present invention, the tissue sample may be in a formalin-fixed paraffin-embedded form or a fresh frozen form.

In one embodiment of the present invention, the number of the patient-specific genomic variants may be 20 to less than 300.

In one embodiment of the present invention, the blood-derived sample may be cell-free DNA or the genome of peripheral blood cells.

In one embodiment of the present invention, the clinically actionable target may be at least one gene selected from the group consisting of ABL1, AKT1, ALK, APC, BRAF, BRCA1, BRCA2, BTK, CDKN2A, CTNNB1, EGFR, ERBB2, EZH2, FGFR2, FGFR3, FLT3, GNAS, HRAS, IDH1, IDH2, KIT, KRAS, MET, MTOR, MYC, MYCN, NRAS, NTRK1, NTRK3, PGFRA, PIK3CA, PTEN, and TP53.

In one embodiment of the present invention, the patient-specific genomic variant may be a variant included in an exon of a target gene.

In one embodiment of the present invention, step (c) may comprise determining that cancer has recurred, when one or more ctDNA molecules are detected in the blood-derived sample from the patient of interest, that is, when two or more patient-specific genomic variants and/or clinically actionable targets are detected.

Another object of the present invention is to provide a method for providing information for predicting cancer recurrence and prognosis, the method comprising steps of: a) obtaining patient-specific genomic variant information from a tissue sample from a patient of interest; b) generating a patient-specific panel by combining the obtained patient-specific genomic variant information and clinically actionable target information; and c) comparing information of the patient-specific panel with sequencing information of a blood-derived sample from the patient of interest, thereby determining whether the patient of interest has cancer recurrence.

### Advantageous Effects

According to the method for predicting cancer recurrence according to one embodiment of the present invention, it is possible to predict cancer recurrence with increased sensitivity and accuracy.

### Brief Description of Drawings

FIG. 1 is a schematic view showing a method for providing information for predicting cancer recurrence and prognosis according to one embodiment of the present invention.
FIG. 2 is a list showing clinically applicable target information included in a bespoke panel generated according to one embodiment of the present invention.
FIG. 3 is a schematic view showing a method of detecting patient-specific tumor tissue variants from tumor tissue whole-exome sequencing data or whole-length genome sequencing data.
FIG. 4 is a flow chart showing a series of analysis processes of obtaining a raw variant call from a tumor tissue sample.
FIG. 5 is a flow chart showing a process of removing false positives and selecting tumor patient-specific variants.
FIG. 6 is a schematic view showing tiers for screening patient-specific variants.
FIGS. 7 and 8 are graphs showing the results of evaluating the analytical performance of a platform using a bespoke panel generated according to one embodiment of the present invention.
FIG. 9 is a graph showing the results of evaluating the clinical performance of a platform using a bespoke panel generated according to one embodiment of the present invention.

### Best Mode

One aspect of the present invention provides a method for providing information for predicting cancer recurrence and prognosis, which is performed by a system for providing information for predicting cancer recurrence and prognosis, the method comprising steps of: a) obtaining patient-specific genomic variant information from a tissue sample from a patient of interest; b) generating a patient-specific panel by combining the obtained patient-specific genomic variant information and clinically actionable target information; and c) comparing information of the patient-specific panel with sequencing information of a blood-derived sample from the patient of interest, thereby determining whether the patient of interest has cancer recurrence.

The method for providing information for predicting cancer recurrence and prognosis according to one embodiment of the present invention is a method for providing information for predicting cancer recurrence and prognosis comprising: analyzing the genome of the tumor tissue obtained from a patient after surgery; selecting patient-specific genomic variants; examining a DNA sample obtained from the blood of the patient visiting the hospital after surgery; and predicting cancer recurrence and prognosis depending on whether the selected patient-specific genomic variants are detected.

According to one embodiment, a patient-specific mutation capture panel (bespoke panel) for enriching and capturing patient-specific variants from blood may be generated in order to predict cancer recurrence and prognosis by the method of the present invention. In the present specification, the "bespoke panel" may be used in the same sense as the "patient-specific panel".

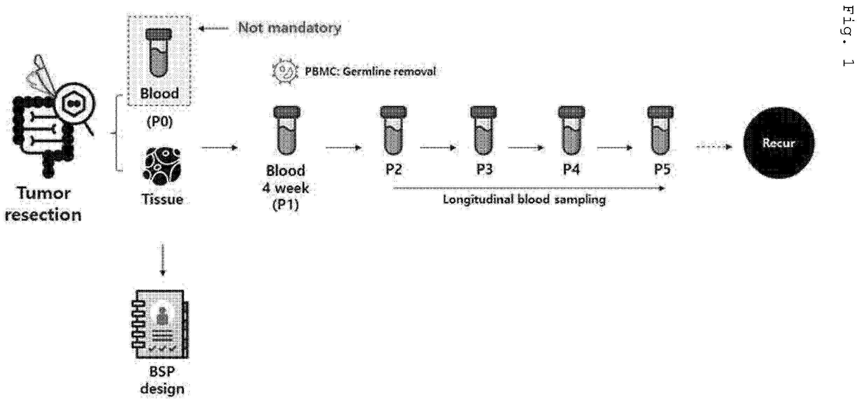

FIG. 1 is a schematic view showing a method for providing information for predicting cancer recurrence and prognosis according to one embodiment of the present invention. In general, when a cancer patient undergoes tumor resection surgery, the tumor tissue and blood from the patient are collected and stored in order to determine whether or not the cancer recurs later. Previously, in order to confirm cancer recurrence, all genomic information obtained from the patient's blood and tissue was used. However, in the method for providing information for predicting cancer recurrence and prognosis according to the present invention, it is possible to predict cancer recurrence using a bespoke panel generated based on only genomic variant information obtained from the tumor tissue from the patient of interest, and thus blood (indicated as PO in FIG. 1) collected immediately after surgery is not mandatory. Therefore, the method according to the present invention has advantages in that it is possible to enroll the patient of interest without a blood sample as a reference, and the number of target samples decreases, which reduces the sequencing cost.

When genomic analysis is performed from a patient's tumor tissue, patient-specific genomic variants are obtained to include all of the patient-specific germline variants from PBMCs without removing them. According to one embodiment, the tissue sample obtained from the tumor tissue may be in a formalin-fixed paraffin-embedded form or a fresh frozen form, without being limited thereto. According to one embodiment, the number of patient-specific genomic variants may be 10 to less than 500, preferably 20 to less than 400, more preferably 20 to less than 300, most preferably 20 to less than 200.

According to one embodiment of the present invention, the DNA sample obtained from the blood of the patient includes cell-free DNA (cfDNA) and genomic DNA (gDNA) of peripheral blood mononuclear cells (PBMCs), without being limited thereto.

In order to predict cancer recurrence according to the present invention, among patient-specific variants, patient-specific germline variants are removed using PBMC gDNA obtained from the blood sample from the patient of interest at time point P1 (the time point of collecting the first blood sample after surgery), and only somatic mutation information for cfDNA is examined. Whether cancer recurs is diagnosed depending on whether circulating tumor DNA (ctDNA) is detected in the patient's blood at time point P1, and when ctDNA is not detected, blood is sampled and tested over time (e.g., P2 to P5).

Only a desired region on the genome among DNA molecules present in the DNA sample from the blood obtained from the patient of interest may be captured and enriched by a hybridization capture method using the generated bespoke panel.

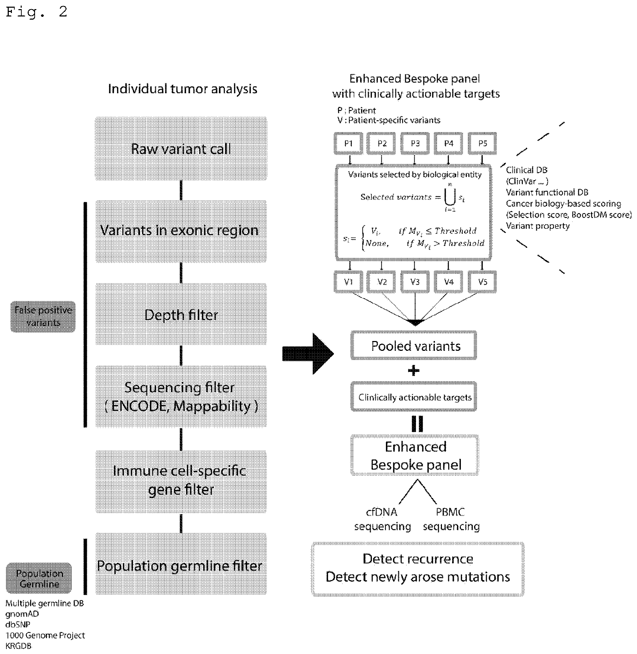

According to one embodiment, the bespoke panel may be generated by combining patient-specific variant information with information for clinically actionable targets, which are regions capable of capturing variants which frequently appear in tumor patient groups or against which drugs may be used directly to treat tumor patients. FIG. 2 is a list showing clinically applicable target information included in a bespoke panel generated according to one embodiment of the present invention. According to one embodiment, the clinically actionable target may be at least one gene selected from the group consisting of ABL1, AKT1, ALK, APC, BRAF, BRCA1, BRCA2, BTK, CDKN2A, CTNNB1, EGFR, ERBB2, EZH2, FGFR2, FGFR3, FLT3, GNAS, HRAS, IDH1, IDH2, KIT, KRAS, MET, MTOR, MYC, MYCN, NRAS, NTRK1, NTRK3, PGFRA, PIK3CA, PTEN, and TP53, without being limited thereto. For example, when all of the targets (33 genes in total) are used, 261 variants present in the genes may be targeted. In FIG. 2, gray shading represents genes required for genomic testing, established by the Korean FDA.

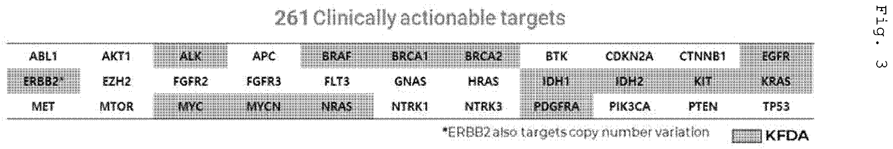

In order to perform genomic analysis of tumor tissue and detect variants, whole-exome sequencing or whole-genome sequencing for the tumor tissue may be used so that sufficient somatic variants are included. An analysis procedure for selecting patient-specific variants through genomic analysis of tumor tissue is shown in FIG. 3. Referring to FIG. 3, starting with a raw variant call, a process of removing false positives is performed. Then, for the variant call from which false positives have been removed, patient-specific variants are selected by combining a database for clinically actionable target information with information on a database reflecting the biological characteristics of the tumor of the patient of interest. For the selected tumor tissue variants, according to the grade of variants, variants from a high grade to the total number of target patient-specific variants are selected.

A bespoke panel according to one embodiment of the present invention may be generated using information on a single patient of interest or generated to include information on a plurality of patients of interest. In addition, since the bespoke panel includes clinically actionable target information, it is possible to provide information on recurrence of previously known cancers and recurrence of cancer specific to the patient of interest.

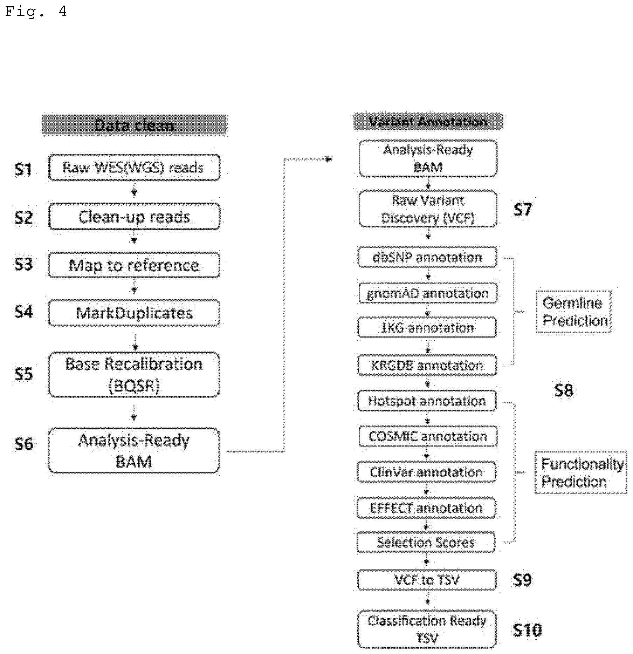

FIG. 4 shows a flow chart for obtaining a raw variant call, which is a starting step in an analysis procedure for selecting patient-specific variants.

The raw variant call is generally obtained by performing a data clean process and a variant annotation process. The sequencing data (S1) obtained through whole-exome sequencing or whole-genome sequencing is sequencing reads in FASTQ format, and is subjected to a data clean-up process (S2) of removing low-quality bases or sequencing adapter sequences used in the sequencing sample preparation process. The sequencing reads subjected to the clean-up process are compared with a reference sequence of the human genome to map positions in the reference sequence (S3). Thereafter, the mapped read data is subjected to a process (S4) of removing duplicate reads generated during the experiment, and subjected to a process (S5) of recalibrating the quality of bases using a statistical method. The format of the analysis-ready data file obtained through the series of processes is BAM (Binary sequence Alignment/Map) format (S6) .

Thereafter, the analysis-ready BAM file is subjected to raw variant discovery (variant call) in the tumor using variant discovery software (e.g., Mutect2). The format of the raw variant call file is variant call format (VCF) (S7). The variants present in the raw variant call file are subjected to variant annotation (S8) including germline prediction and functionality prediction using various databases to create a VCF file. The VCF file is converted to a tab-separated values (TSV) text file (S9), and a classification-ready TSV data file is finally obtained (S10). The database used for the variant annotation may include the following databases whose annotations are disclosed in S8 in FIG. 4:
- somatic variant database
- database containing the clinical usefulness of variants
- database for functions for proteins that variants actually affect
- database that provides scores that consider tumor biology for variants

Thereafter, the variant call with various annotations is used as a raw variant call to remove false positives and select tumor patient-specific variants.

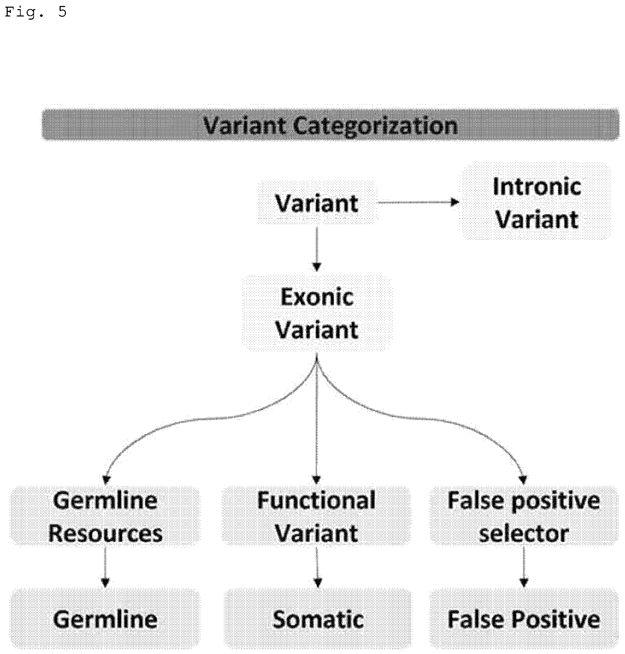

FIG. 5 shows a process of removing false positives and categorizing tumor patient-specific variants. According to one embodiment, in this process, a process of screening only variants corresponding to exons is first performed, thereby removing variants corresponding to introns. That is, the patient-specific genomic variant may be a variant included in an exon of a target gene. In addition, the screening process is followed by a process of removing variants generated in the depth filter due to insufficient reading in the genome sequencing results of tumor tissue, a process of removing variants that occur in regions that are not well sequenced or have abnormal data distribution, which are already known in the human genome, and a process of removing variants corresponding to genes related to immune cells. As a result, variants that are thought to have an effect on tumor development are set as patient-specific variants, which are subjected to a process of selecting variants detected in a clinically useful database, and a process of selecting variants detected in a database using biological characteristics of tumors. Examples of the clinically useful database include, but are not limited to, COSMIC, ClinVar, and hotspot. After selecting only variants corresponding to exons, the variants are categorized into three different classes (germline variants, somatic variants, and false-positive variants) using the variant annotation attached in the previous step.

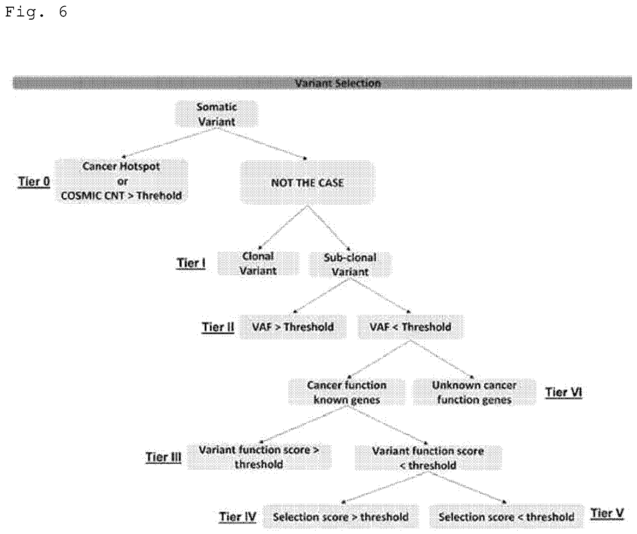

FIG. 6 is a schematic view showing tiers for screening patient-specific variants. Tiers are biological characteristics used to increase the probability of detecting ctDNA in a sample of interest, since cells present in tumor tissue are genetically very heterogeneous. As a result of the previous variant screening and categorization, final patient-specific variants are selected through seven tiers (tier 0 to tier VI) for patient-specific variants corresponding to somatic variants. Description of each tier is as follows.

Tier 0: variants that occur frequently in tumors or have been frequently reported in tumor databases.

Tier I: variants detected in a clonal tumor cell population.

Specifically, these variants are selected from the most dominant cell population, that is, the cell population derived from the cells of cancer that would have occurred first, among the entire tumor cell population. When cancer recurrence occurs, this criterion is used because the probability of recurrence from the primary cancer is high.

Tier II: variants whose variant allele frequency (VAF) passes the desired threshold.

Specifically, although these variants are clonal, they occupy the majority of the tumor population. This criterion is used because there is a high probability of being released into blood at the time of recurrence.

Tier III: variants whose variant function score (Tier I) passes the threshold, among the genes involved in the generation/development of tumors.

Tier IV: variants whose variant function score (Tier II) passes the threshold, among the genes involved in the generation/development of tumors.

Tier V: variants whose variant function score (Tier II) does not pass the threshold, among the genes involved in the generation/development of tumors.

Tier VI: variants found in genes other than those involved in the generation/development of tumors.

Finally, it may be determined that cancer has recurred, when one or more ctDNA molecules are detected in the blood-derived sample from the patient of interest, that is, when two or more patient-specific genomic variants and/or clinically actionable targets are detected.

Another object of the present invention is to provide a method for providing information for predicting cancer recurrence and prognosis, the method comprising steps of: a) obtaining patient-specific genomic variant information from a tissue sample from a patient of interest; b) generating a patient-specific panel by combining the obtained patient-specific genomic variant information and clinically actionable target information; and c) comparing information of the patient-specific panel with sequencing information of a blood-derived sample from the patient of interest, thereby determining whether the patient of interest has cancer recurrence.

Step a) is a step of obtaining patient-specific genomic variant information from a tissue sample from a patient of interest, and may be performed through whole-exome sequencing or whole-genome sequencing.

Step b) is a step of generating a patient-specific panel by combining the obtained patient-specific genomic variant information and clinically actionable target information.

Step c) is a step of comparing information of the patient-specific panel with sequencing information of a blood-derived sample from the patient of interest, thereby determining whether the patient of interest has cancer recurrence. This step may be performed through the criterion for determining that cancer has recurred, when two or more patient-specific genomic variants and/or clinically actionable targets are detected.

### Mode for Invention

Hereinafter, one or more embodiments will be described in more detail with reference to examples. However, these examples are for explaining one or more embodiments in detail, and the scope of the present invention is not limited to these examples.

### Example 1. Tissue gDNA extraction and bespoke panel generation

The tumor tissue used in this Example was in a formalin-fixed paraffin-embedded (FFPE) form or a fresh frozen (FF) form. From the sample in the FFPE form, FFPE gDNA was extracted using an FFPE gDNA extraction kit (Promega, USA), and from the sample in the FF form, FF gDNA was extracted using a tissue gDNA extraction kit (Promega, USA). Then, the extracted FFPE gDNA was quality-checked (QC) using a TapeStation System (Agilent, USA), and the FF gDNA was quality-checked using a Qubit fluorometer (Thermofisher Scientific, USA) and Nanodrop (Thermofisher Scientific, USA). Each of the extracted gDNAs was sheared to a desired size (main peak: 180 to 220 bp) using a sonicator or enzyme and quality-checked using a TapeStation System (Agilent, USA). At this time, in the case of the sheared FFPE gDNA, the pretreatment was completed by repairing the damaged DNA using an FFPE DNA repair kit (New England Biolabs, USA).

Next, each of the extracted gDNAs was subjected to the NGS DNA library preparation process using xGen Prism kit (Integrated Device Technology, USA). The library preparation process consisted of end repair, adapter ligation, and PCR amplification. The prepared DNA libraries were subjected to whole-exome sequencing (WES) using a whole-exome enrichment kit (Agilent, USA). In this case, a plurality of samples may be pooled or a single sample may be used. Then, the completed gDNA whole-exome enriched libraries were quality-checked using a TapeStation System (Agilent, USA). Thereafter, each sample was mixed according to the desired amount of NGS data for the gDNA whole-exome enriched library and subjected to NGS using Illumina's Nextseq or Novaseq system. Next, an NGS panel was designed based on variants generated in tissue in the NGS data produced from the library, thereby generating a patient-specific bespoke panel.

### Example 2. Method of predicting cancer recurrence using blood gDNA extraction and bespoke panel

Plasma and peripheral blood mononuclear cells (PBMCs) were isolated from the blood from the patient of interest, and cfDNA was extracted from the plasma using a cfDNA extraction kit (Promega, USA). The extracted cfDNA was quality-checked using a TapeStation System (Agilent, USA). At this time, samples continuously obtained from one patient of interest were labeled as P0 (the time point of surgery) and P1, P2, etc. (subsequent sequential time points). In addition, PBMC gDNA was extracted from the isolated PBMCs using a Blood gDNA extraction kit (Promega, USA), and quality-checked using a Qubit fluorometer (Thermofisher Scientific, USA) and Nanodrop (Thermofisher Scientific, USA). The quality-checked PBMC gDNA was sheared to a desired size (main peak: 180 to 220 bp) using a sonicator or enzyme. The prepared cfDNA and/or sheared PBMC gDNA was subjected to the NGS DNA library preparation process. The library preparation process consisted of end repair, adapter ligation, and PCR amplification. The prepared DNA library was subjected to targeted enrichment for the patient of the interest using the bespoke panel generated in Example 1. The completed target-enriched library was quality-checked using a TapeStation System (Agilent, USA), and then each sample was mixed according to the desired amount of NGS data for the bespoke panel enriched library and subjected to NGS using Illumina's Novaseq system.

### Example 3. Evaluation of analytical performance of cancer recurrence prediction platform using bespoke panel

To evaluate the analytical performance of the cancer recurrence prediction platform according to one embodiment of the present invention, each of two different B-lymphocytes (NA12891 and NA12892) having known genotypes were mixed at 1%, 0.5%, 0.1%, 0.05%, 0.01%, 0.005%, 0.001%, and 0% (control) of the total, and the experimental group, which was mixed at a small ratio, was mimicked as a mixed state of tumor, and the mixed ratio was mimicked as the ratio of ctDNA. At this time, as targets for monitoring, among the known SNP markers in each cell line disclosed through the HapMap project, only different markers (e.g., one cell line is a reference to a human reference, and another cell line is not) were selected and used. The amount of DNA used was 40 ng.

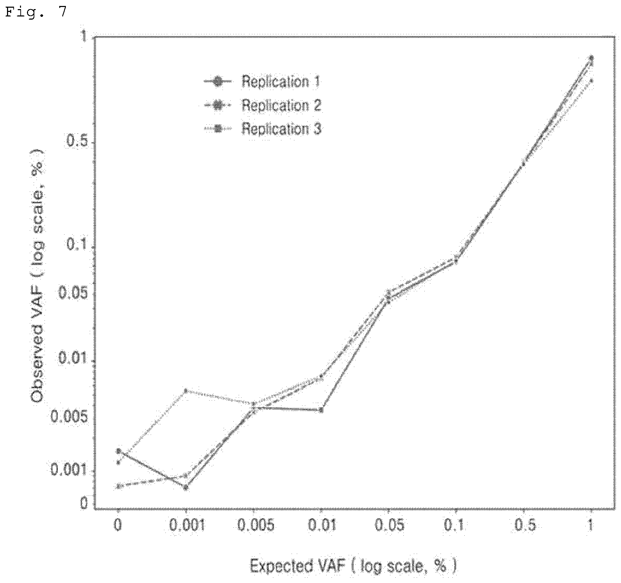

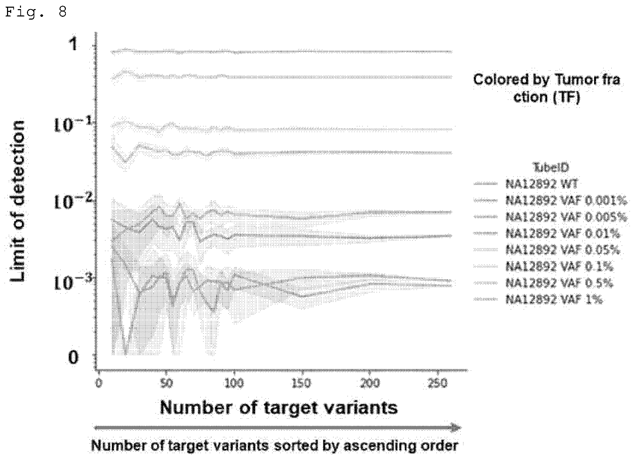

As a result of examining a total of three replicates, as shown in FIG. 7, it was confirmed that detection was possible at 0.005% or more compared to 0% (control). FIG. 8 is a graph showing the detection limit as a function of the number of target variants, where the x-axis indicates the number of monitored variants, and the y-axis indicates the fraction of detected ctDNA. Random sampling was performed 10 times so that the number of variants was as much as the corresponding number from the maximum number (257). In the graph of FIG. 8, the shadowed portion means the observed range, and it can be confirmed that, as the number of variants increases, a low fraction of ctDNA can be detected more stably. That is, it was confirmed that, when the number of somatic variants was 100 or more, up to 0.005% could be stably detected.

### Example 4. Evaluation of clinical performance of cancer recurrence prediction platform using bespoke panel

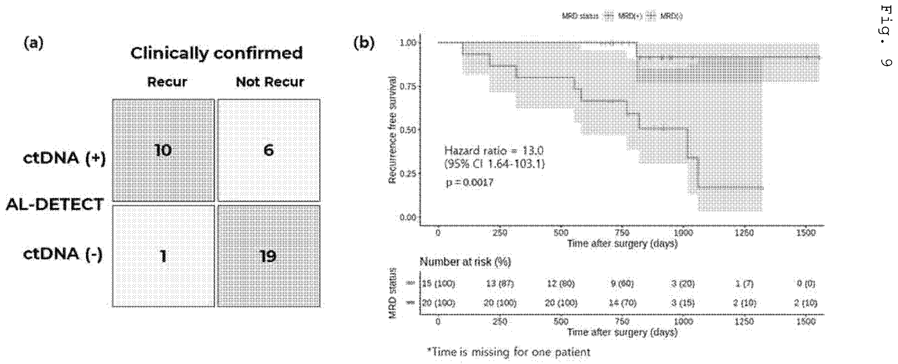

To evaluate the clinical performance of the cancer recurrence prediction platform according to one embodiment of the present invention, evaluation was conducted on 36 patients, which are part of a cohort of 100 colorectal cancer patients. It was confirmed clinically that colorectal cancer recurred in 11 of these patients (FIG. 9(a)). As a result of clinical performance evaluation, sensitivity was 91% and negative predictive value (NPV) was 95%. As a result of analyzing survival depending on the state of minimal residual disease (MRD) using the method for predicting cancer recurrence according to the present invention, it could be confirmed that there was a statistically significant (p<0.05) difference in survival between the recurrence group and the non-recurrence group (one of 11 recurred patients was excluded from survival analysis because the time of recurrence was not clear). In addition, it could be confirmed that the group predicted to recur was 13 times more hazardous (hazard ratio = 13.0) than the non-predicted group (FIG. 9(b)).

So far, the present invention has been described with reference to the preferred embodiments. Those skilled in the art will appreciate that the present invention can be implemented in modified forms without departing from the essential features of the present invention. Therefore, the disclosed embodiments should be considered in descriptive sense only and not for purposes of limitation. Therefore, the scope of the present invention is defined not by the detailed description of the present invention but by the appended claims, and all differences within a range equivalent to the scope of the appended claims should be construed as being included in the present invention.

## Claims

1. A method for providing information for predicting cancer recurrence and prognosis, which is performed by a system for providing information for predicting cancer recurrence and prognosis, the method comprising steps of:
a) obtaining patient-specific genomic variant information from a tissue sample from a patient of interest;
b) generating a patient-specific panel by combining the obtained patient-specific genomic variant information and clinically actionable target information; and
c) comparing information of the patient-specific panel with sequencing information of a blood-derived sample from the patient of interest, thereby determining whether the patient of interest has cancer recurrence.

2. The method according to claim 1, wherein the tissue sample is in a formalin-fixed paraffin-embedded form or a fresh frozen form.

3. The method according to claim 1, wherein the number of the patient-specific genomic variants is 20 to less than 300.

4. The method according to claim 1, wherein the blood-derived sample is cell-free DNA or the genome of peripheral blood cells.

5. The method according to claim 1, wherein the clinically actionable target is at least one gene selected from the group consisting of ABL1, AKT1, ALK, APC, BRAF, BRCA1, BRCA2, BTK, CDKN2A, CTNNB1, EGFR, ERBB2, EZH2, FGFR2, FGFR3, FLT3, GNAS, HRAS, IDH1, IDH2, KIT, KRAS, MET, MTOR, MYC, MYCN, NRAS, NTRK1, NTRK3, PGFRA, PIK3CA, PTEN, and TP53.

6. The method according to claim 1, wherein the patient-specific genomic variant is a variant included in an exon of a target gene.

7. The method according to claim 1, wherein step (c) comprises determining that cancer has recurred, when one or more ctDNA molecules are detected in the blood-derived sample from the patient of interest, that is, when two or more patient-specific genomic variants and/or clinically actionable targets are detected.

8. A method for providing information for predicting cancer recurrence and prognosis, the method comprising steps of:
a) obtaining patient-specific genomic variant information from a tissue sample from a patient of interest;
b) generating a patient-specific panel by combining the obtained patient-specific genomic variant information and clinically actionable target information; and
c) comparing information of the patient-specific panel with sequencing information of a blood-derived sample from the patient of interest, thereby determining whether the patient of interest has cancer recurrence.
